(19) Europäisches Patentamt / European Patent Office / Office européen des brevets

(11) **EP 4 678 166 A1**

(12) **EUROPEAN PATENT APPLICATION**
published in accordance with Art. 153(4) EPC

(43) Date of publication:
**14.01.2026 Bulletin 2026/03**

(21) Application number: 24769789.9

(22) Date of filing: **05.03.2024**

(51) International Patent Classification (IPC):
$A61K\ 9/19^{(2006.01)}$    $A61K\ 41/00^{(2020.01)}$
$A61K\ 47/26^{(2006.01)}$    $A61K\ 47/02^{(2006.01)}$
$A61P\ 35/00^{(2006.01)}$

(52) Cooperative Patent Classification (CPC):
A61J 3/02; A61K 9/19; A61K 41/00; A61K 47/02;
A61K 47/26; A61P 35/00

(86) International application number:
**PCT/CN2024/080013**

(87) International publication number:
**WO 2024/188095 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2023 CN 202310247560**
**30.01.2024 CN 202410128924**

(71) Applicant: Neuboron Bio-Scitech Co., Ltd.
**Xiamen City, Fujian Prov. (CN)**

(72) Inventors:
• **WANG, Wei**
**Nanjing, Jiangsu 211112 (CN)**
• **ZHOU, Ping**
**Nanjing, Jiangsu 211112 (CN)**
• **BIAN, Ya**
**Nanjing, Jiangsu 211112 (CN)**
• **CHEN, Lamei**
**Nanjing, Jiangsu 211112 (CN)**
• **LIU, Yuanhao**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(54) **PREPARATION METHOD FOR LARGE-SIZE BORONOPHENYLALANINE LYOPHILIZED POWDER FOR INJECTION AND LYOPHILIZED POWDER FOR INJECTION**

(57) The present invention relates to the technical field of medicine and provides a preparation method for large-specification dihydroxyboronphenylalanine lyophilized powder for injection. The preparation method for large-specification dihydroxyboronphenylalanine lyophilized powder for injection includes: mixing dihydroxyboronphenylalanine, a polyol, a base, and part of a solvent to obtain a first mixed solution; adjusting the pH of the first mixed solution by using a pH regulator, and adding the remaining part of the solvent to obtain a second mixed solution, wherein the concentration of dihydroxyboronphenylalanine in the second mixed solution is 160-250 mg/mL; and aliquoting the second mixed solution, followed by lyophilization, where the lyophilization includes pre-freezing, sublimation drying, and desorption drying. By the preparation method of the present invention, stable large-specification dihydroxyboronphenylalanine lyophilized powder for injection can be prepared. The method of the present invention has the characteristics of stable process and reasonable lyophilization time, thereby greatly reducing the lyophilization costs.

**Description**

**TECHNICAL FIELD**

The present invention relates to the technical field of medicine, and specifically to a preparation method for large-specification dihydroxyboronphenylalanine lyophilized powder for injection and lyophilized powder for injection prepared by the method.

**BACKGROUND**

L-BPA is a boron carrier used in boron neutron capture therapy, which requires 109 boron atoms in tumor cells. However, due to the low boron loading of L-BPA and limited targeting, the dosage is very large, reaching 100-500 mg/kg. Based on an adult weight of 60 kg, the single dose for a single person is 30 g. L-BPA-sugar alcohol solution is often used in L-BPA formulations. However, L-BPA-sugar alcohol solution is sensitive to temperature and difficult to store, so L-BPA formulations are generally prepared in lyophilized form.

Conventional lyophilized products are generally only suitable for products with specifications less than 1 g due to the limitations of lyophilization concentration and liquid volume. Low-specification lyophilized products have the following disadvantages for L-BPA with a single dose of 30 g. Using multiple bottles of products for a single injection and opening and dissolving each bottle separately not only make the operation cumbersome but also increase the risk of sterility. Using multiple bottles for a single injection increases the amount of packaging materials, which not only raises costs but also imposes a burden on the environment. However, large-specification lyophilized formulations are not conducive to water sublimation due to their high solid content, generally require a long time and high costs for lyophilization, and may collapse in various forms when melting due to a temperature rise or when the ice crystal support structure is removed by drying, making it difficult to form a stable dosage form, affecting dissolution, product stability, and other quality indicators. It is difficult to develop large-specification lyophilized formulations, and there are almost no lyophilized powder formulations on the market that exceed 3 g in specifications.

Patent Publication No. CN103100094B discloses a lyophilization process for L-BPA, in which the L-BPA content in a single vial is as low as 30 mg, which cannot meet the clinical drug administration requirements. Patent Publication No. CN113546048A discloses a method for reducing the osmotic pressure of a boron carrier injection, in which nanofiltration technology is used to reduce the osmotic pressure of the solution before lyophilization, which is complicated to operate; and the lyophilization process of the 3 g/vial specification includes an additional annealing step, and the lyophilization time is as long as 109 hours, resulting in long time, high power consumption and high costs.

Therefore, there is an urgent need to provide a preparation method for large-specification dihydroxyboronphenylalanine lyophilized powder for injection, to prepare large-specification dihydroxyboronphenylalanine lyophilized powder for injection, thereby solving the technical problems in the prior art.

**SUMMARY**

An objective of the present invention is to provide a preparation method for large-specification dihydroxyboronphenylalanine lyophilized powder for injection, by which stable large-specification dihydroxyboronphenylalanine lyophilized powder for injection can be prepared. The method of the present invention has the characteristics of stable process and reasonable lyophilization time.

To achieve the above objective, the present invention provides the following technical solutions.

According to a first aspect of the present invention, a preparation method for large-specification dihydroxyboronphenylalanine lyophilized powder for injection is provided, including:

mixing dihydroxyboronphenylalanine, a polyol, a base, and part of a solvent to obtain a first mixed solution;
adjusting the pH of the first mixed solution to 7.5-8.5 by using a pH regulator, and adding the remaining part of the solvent to obtain a second mixed solution, where the concentration of dihydroxyboronphenylalanine in the second mixed solution is 160-250 mg/mL; and
aliquoting the second mixed solution, followed by lyophilization, where the lyophilization includes pre-freezing, sublimation drying, and desorption drying.

In some embodiments of the present invention, the dihydroxyboronphenylalanine includes at least one of 2-dihydroxyboron-phenylalanine, 3-dihydroxyboron-phenylalanine, or 4-dihydroxyboron-phenylalanine (BPA). For example, the dihydroxyboronphenylalanine may be 4-dihydroxyboron-phenylalanine (BPA), e.g., L-BPA or D-BPA. For example, the dihydroxyboronphenylalanine may be 2-dihydroxyboron-phenylalanine and/or 3-dihydroxyboron-phenylalanine.

In some embodiments of the present invention, the concentration of dihydroxyboronphenylalanine in the second mixed solution may be 160 mg/mL, 170 mg/mL, 180 mg/mL, 190 mg/mL, 200 mg/mL, 210 mg/mL, 220 mg/mL, 230 mg/mL, 240 mg/mL, or 250 mg/mL. The concentration of lyophilization solution used in lyophilization of the dihydroxyboronphenylalanine lyophilized powder formulation is generally low, and the concentration of lyophilization solution that has been disclosed in the prior art basically does not exceed 100 mg/mL. The product concentration greatly affects the shaping and redissolubility of the lyophilized powder.

In some embodiments of the present invention, conditions of the pre-freezing include: a temperature of -60°C to -20°C and atmospheric pressure. Preferably, the temperature of the pre-freezing is -50°C to -40°C. For example, the temperature of the pre-freezing is -60°C, -59°C, -57°C, -55°C, -52°C, -50°C, -49°C, -48°C, -47°C, -46°C, -45°C, -44°C, -43°C, -42°C, -41°C, -40°C, -38°C, -35°C, -33°C, -30°C, -28°C, -26°C, -24°C, -22°C, or -20°C. The atmospheric pressure of the pre-freezing is generally 1 atmosphere.

In some embodiments of the present invention, the time of the pre-freezing is 2-10 hours. Preferably, the time of the pre-freezing is 2-5 hours. For example, the time of the pre-freezing may be 2 hours, 3 hours, 4 hours, 5 hours, 6 hours, 7 hours, 8 hours, 9 hours, or 10 hours.

In some embodiments of the present invention, the pre-freezing is: maintaining the temperature at -50°C for 2-10 hours. The pre-freezing process of the present invention does not include an annealing step, so the lyophilization time is shortened, and the process costs and operation difficulty are reduced.

In the embodiments of the present invention, the pre-freezing stage of the preparation method requires a short time, and the pre-freezing costs are effectively reduced.

In some embodiments of the present invention, conditions of the sublimation drying include: a temperature of -10°C to -2°C and a pressure of 10-20 pa. For example, the temperature of the sublimation drying may be -10°C, -9°C, -8°C, -7°C, -6°C, -5°C, -4°C, -3°C, or -2°C. For example, the pressure of the sublimation drying is 10 pa, 11 pa, 12 pa, 13 pa, 14 pa, 15 pa, 16 pa, 17 pa, 18 pa, 19 pa, or 20 pa. After the above pre-freezing stage, sublimation drying of the high-concentration dihydroxyboronphenylalanine solution can be realized by one sublimation in the sublimation drying stage of the present invention, to effectively remove water.

In some embodiments of the present invention, the time of the sublimation drying is 24-54 hours. For example, the time of the sublimation drying may be 24 hours, 25 hours, 26 hours, 27 hours, 30 hours, 32 hours, 35 hours, 37 hours, 39 hours, 40 hours, 42 hours, 45 hours, 47 hours, 49 hours, 50 hours, 53 hours, or 54 hours.

In some embodiments of the present invention, the sublimation drying is: maintaining the temperature at -5°C for 24-54 hours. Further, the sublimation drying is: maintaining the temperature at -5°C and the pressure at 10-20 pa for 24-54 hours. The sublimation drying process of the present invention does not require an annealing step, the primary drying temperature is increased, the lyophilization time is shortened, the drying effect is realized, and the process costs and operation difficulty are reduced.

In the preparation method of the embodiments of the present invention, the combination of the high concentration of dihydroxyboronphenylalanine in the mixed solution and the treatment temperature in the sublimation drying stage in the present invention can prevent the collapse of the lyophilized product. It can be seen that at the sublimation drying temperature of the present invention, not only a lyophilized powder with loose appearance can be obtained, but also the efficiency of sublimation drying can be improved, thereby reducing the drying time and the drying costs.

In some embodiments of the present invention, conditions of the desorption drying include: a temperature of 25°C to 35°C and a pressure of 10-20 pa. For example, the temperature of the desorption drying may be 25°C, 26°C, 27°C, 28°C, 29°C, 30°C, 31°C, 32°C, 33°C, 34°C, or 35°C. For example, the pressure of the desorption drying may be 10 pa, 11 pa, 12 pa, 13 pa, 14 pa, 15 pa, 16 pa, 17 pa, 18 pa, 19 pa, or 20 pa.

In some embodiments of the present invention, the time of the desorption drying is 5-15 hours. Preferably, the time of the desorption drying is 12-14 hours. For example, the time of the desorption drying may be 5 hours, 6 hours, 7 hours, 8 hours, 9 hours, 10 hours, 11 hours, 12 hours, 12.5 hours, 13 hours, 13.5 hours, 14 hours, or 15 hours.

In the preparation method of the present invention, the solution can be brought into a frozen state through the pre-freezing, which is conducive to the subsequent drying operation. After the sublimation drying of the present invention, the liquid level reaches the bottom of the vial, and lyophilized powder with loose appearance can be obtained through the desorption drying.

In some embodiments of the present invention, the mass ratio of the dihydroxyboronphenylalanine to the polyol is 1:1 to 1:1.3. For example, the mass ratio of the dihydroxyboronphenylalanine to the polyol may be 1:1, 1:1.2, or 1:1.3.

The polyol is selected from at least one of fructose, sorbitol, mannitol, erythritol, maltitol, lactitol, or xylitol.

In some embodiments of the present invention, the base is selected from at least one of sodium hydroxide, magnesium hydroxide, potassium hydroxide, or calcium hydroxide.

In some embodiments of the present invention, the pH regulator is selected from at least one of hydrochloric acid, acetic acid, phosphoric acid, nitric acid, or sulfuric acid.

In some embodiments of the present invention, the solvent is water for injection.

In some embodiments of the present invention, the second mixed solution is subjected to sterilization treatment before the lyophilization; and the sterilization treatment includes: sterilizing the second mixed solution by filtration through a microfiltration membrane. Specifically, the second mixed solution is sterilized by filtration through 0.45 micron and 0.22 micron microfiltration membranes.

In some embodiments of the present invention, the liquid height is 15-30 mm after aliquoting. Specifically, after the aliquoting, the liquid height in the vial may be 15 mm, 17 mm, 19 mm, 20 mm, 22 mm, 24 mm, 26 mm, 28 mm, or 30 mm.

In some embodiments of the present invention, the liquid volume in each vial after the aliquoting is 15-30 mL. For example, the liquid volume in each vial after the aliquoting may be 15 mL, 16 mL, 18 mL, 20 mL, 22 mL, 25 mL, 26 mL, 28 mL, or 30 mL.

In some embodiments of the present invention, the liquid height in a vial containing 10 mL of liquid is approximately 10 mm. In other words, in the following examples, the liquid height can be calculated according to the liquid volume after aliquoting.

In some embodiments of the present invention, each vial obtained after the aliquoting contains 3-7.5 grams of dihydroxyboronphenylalanine. For example, each vial obtained after the aliquoting may contain 3 grams, 3.5 grams, 4 grams, 5 grams, 6 grams, 7 grams, or 7.5 grams of dihydroxyboronphenylalanine.

According to a second aspect of the present invention, a lyophilized powder for injection is provided, which is prepared by the preparation method according to the first aspect of the present invention. The lyophilized powder for injection contains dihydroxyboronphenylalanine. Further, each vial of the lyophilized powder for injection contains 3-7.5 grams of dihydroxyboronphenylalanine.

It should be understood that within the scope of the present invention, the above technical features of the present invention and the technical features to be described in detail below (e.g., in examples) can be combined with each other to form a new or preferred technical solution. For brevity of description, the details will not be repeated herein.

Pre-freezing: the product can be sublimation dried only after pre-freezing. The pre-freezing process is not only to maintain the main properties of the product, but is also to obtain a lyophilized product with a reasonable structure to facilitate the sublimation of moisture. Pre-freezing is a very important step in the lyophilization process. If the temperature is too high, the product cannot be frozen, and the formation of bubbles and liquid spraying will occur during subsequent sublimation, resulting in a loss of the product. If the temperature is too low, the freezing time will be long, especially for large-specification formulations.

Sublimation drying: also known as primary drying. The sublimation stage of a lyophilized product is the most critical and problem-prone stage which requires stringent temperature control. The temperature of sublimation drying should be lower than the collapse temperature or the maximum allowable temperature of the product. A too low sublimation drying temperature will reduce the drying efficiency and increase the drying time, making it difficult to obtain a dry and loose product in a short time.

Desorption drying: also called secondary drying. The first-stage drying removes water in the form of ice crystals, but for adsorbed water with high adsorption energy, sufficient energy needs to be provided in order to realize the desorption of water.

The present invention has the following beneficial effects.

The preparation method of the large-specification dihydroxyboronphenylalanine lyophilized powder for injection simplifies operation and reduces sterility risks and production costs, and each vial of the prepared lyophilized powder for injection has high content of dihydroxyboronphenylalanine, so the number of vials to be used is reduced. In addition, the lyophilization time of the method of the present invention is short, so the lyophilization costs can be reduced.

The preparation method of the present invention features convenient operation and does not require an annealing step, and the primary drying temperature is increased, so the lyophilization time is shortened, and the process costs and operation difficulty are reduced.

## BRIEF DESCRIPTION OF THE DRAWINGS

The technical solutions and other beneficial effects of the present application will be apparent from the following detailed description of the specific embodiments of the present application with reference to the accompanying drawings.

FIG. 1 is an appearance diagram of a lyophilized powder of Example 1 of the present invention.
FIG. 2 is an appearance diagram of a lyophilized powder of Example 2 of the present invention.
FIG. 3 is an appearance diagram of a lyophilized powder of Example 3 of the present invention.
FIG. 4 is an appearance diagram of a lyophilized powder of Comparative Example 1.

## DETAILED DESCRIPTION

The present disclosure will be further described below through specific examples. It is to be understood that the following description is merely the most preferred embodiments of the present invention and should not be construed as limiting the scope of protection of the present invention. On the basis of a full understanding of the present invention, the experimental methods in the following examples for which specific conditions are not specified are generally carried out under conventional conditions or under conditions recommended by manufacturers. Those skilled in the art may make non-essential changes to the technical solutions of the present invention, and such changes should be construed as falling within the scope of protection of the present invention. In the specification, claims, and accompanying drawings of the present invention, the terms such as "first", "second", and "third", "fourth" (if any) are intended to distinguish between similar objects but do not necessarily indicate a specific order or sequence. It should be understood that objects thus described may be interchangeable in appropriate circumstances. In addition, the terms "include," "comprise," "has," and any variants thereof, are intended to cover a non-exclusive inclusion.

## Example 1

This example provided a preparation method for large-specification dihydroxyboronphenylalanine lyophilized powder for injection, including the following steps.

A prescription was prepared as follows.

| Material name | Type | Amount |
|---|---|---|
| BPA | Raw material | 96 g |
| Fructose | Co-solvent | 110 g |
| Hydrochloric acid | pH regulator | Appropriate amount |
| Sodium hydroxide | Base | Appropriate amount |
| Water for injection | Solvent | 600 mL |

The preparation process was as follows.

In S1, the prescribed amount of BPA and the prescribed amount of fructose were weighed and added to 300 mL of water for injection.

In S2, an appropriate amount of sodium hydroxide was added, followed by stirring until the solution was clear.

In S3, hydrochloric acid was added to adjust the pH to 7.5-8.5.

In S4, water was added to make the volume to 600 mL to obtain a solution containing 160 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 4 g per vial.

In this example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

## Example 2

This example provided a preparation method for large-specification dihydroxyboronphenylalanine lyophilized powder for injection, including the following steps.

A prescription was prepared as follows.

| Material name | Function | Amount |
|---|---|---|
| BPA | Raw material | 120 g |
| Fructose | Co-solvent | 150 g |
| Hydrochloric acid | pH regulator | Appropriate amount |
| Sodium hydroxide | Base | Appropriate amount |
| Water for injection | Solvent | 600 mL |

The preparation process was as follows.

In S1, the prescribed amount of BPA and the prescribed amount of fructose were weighed and added to 300 mL of water for injection.

In S2, an appropriate amount of sodium hydroxide was added, followed by stirring until the solution was clear.

In S3, hydrochloric acid was added to adjust the pH to 7.5-8.5.

In S4, water was added to make the volume to 600 mL to obtain a solution containing 200 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 5 g per vial.

In this example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

### Example 3

This example provided a preparation method for large-specification dihydroxyboronphenylalanine lyophilized powder for injection, including the following steps.

A prescription was prepared as follows.

| Material name | Function | Amount |
|---|---|---|
| BPA | Raw material | 120 g |
| Fructose | Co-solvent | 150 g |
| Hydrochloric acid | pH regulator | Appropriate amount |
| Sodium hydroxide | Base | Appropriate amount |
| Water for injection | Solvent | 600 mL |

The preparation process was as follows.

In S1, the prescribed amount of BPA and the prescribed amount of fructose were weighed and added to 300 mL of water for injection.

In S2, an appropriate amount of sodium hydroxide was added, followed by stirring until the solution was clear.

In S3, hydrochloric acid was added to adjust the pH to 7.5-8.5.

In S4, water was added to make the volume to 600 mL to obtain a solution containing 200 mg/mL of BPA.

In S5, the solution was sterilized by filtration through 0.45 micron and 0.22 micron filter membranes to obtain an intermediate solution.

In S6, filling was performed, i.e., 30 mL of the intermediate solution was added to a vial.

In S7, lyophilization was performed to obtain a product, where the content of the BPA in the product was 6 g per vial.

In this example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

### Example 4

Example 4 provided a preparation method for large-specification dihydroxyboronphenylalanine lyophilized powder for injection, which was different from Example 1 in that the BPA concentration in Example 4 was 250 mg/mL, and the BPA content in each vial after aliquoting was 6.25 grams, as shown in Table 3. The other conditions were substantially the same as those in Embodiment 1.

### Example 5

Example 5 provided a preparation method for large-specification dihydroxyboronphenylalanine lyophilized powder for injection, which was different from Example 1 in that the liquid volume per vial in Example 5 was 15 mL, and the BPA content in each vial after aliquoting was 2.4 grams, as shown in Table 3. The other conditions were substantially the same as those in Embodiment 1.

**Examples 6-9**

Examples 6 to 9 provided a preparation method for large-specification dihydroxyboronphenylalanine lyophilized powder for injection, which was different from Example 1 in that the pre-freezing temperatures in Examples 6-9 were respectively -60°C, -40°C, -30°C, and -20°C, as shown in Table 3. The other conditions were substantially the same as those in Embodiment 1.

**Examples 10-11**

Examples 10 to 11 provided a preparation method for large-specification dihydroxyboronphenylalanine lyophilized powder for injection, which was different from Example 1 in that the pre-freezing respectively lasted for 2 h and 10 h in Examples 10-11, as shown in Table 3. The other conditions were substantially the same as those in Embodiment 1.

**Examples 12-13**

Examples 12 to 13 provided a preparation method for large-specification dihydroxyboronphenylalanine lyophilized powder for injection, which was different from Example 1 in that the sublimation drying temperatures in Examples 12-13 were respectively -10°C and -2°C, as shown in Table 3. The other conditions were substantially the same as those in Embodiment 1.

**Examples 14-16**

Examples 14 to 16 provided a preparation method for large-specification dihydroxyboronphenylalanine lyophilized powder for injection, which was different from Example 1 in that the sublimation drying respectively lasted for 24 h, 54 h, and 30 h in Examples 14-16, as shown in Table 3. The other conditions were substantially the same as those in Embodiment 1.

**Examples 17-18**

Examples 17 to 18 provided a preparation method for large-specification dihydroxyboronphenylalanine lyophilized powder for injection, which was different from Example 1 in that the desorption drying temperatures in Examples 17-18 were respectively 25°C and 35°C, as shown in Table 3. The other conditions were substantially the same as those in Embodiment 1.

**Examples 19-20**

Examples 19 to 20 provided a preparation method for large-specification dihydroxyboronphenylalanine lyophilized powder for injection, which was different from Example 1 in that the desorption drying respectively lasted for 5 h and 14 h in Examples 19-20, as shown in Table 3. The other conditions were substantially the same as those in Embodiment 1.

**Comparative Example 1**

Comparative Example 1 provided a preparation method for dihydroxyboronphenylalanine lyophilized powder for injection, including the following steps.
In S1, BPA and fructose were weighed and added to water for injection.
In S2, an appropriate amount of sodium hydroxide was added, followed by stirring until the solution was clear.
In S3, hydrochloric acid was added to adjust the pH to 7.5-8.5.
In S4, water was added to make the volume to obtain a solution containing 160 mg/mL of BPA.
In S5, the solution was sterilized by filtration through 0.45 micron and 0.22 micron filter membranes to obtain an intermediate solution.
In S6, filling was performed, i.e., 25 mL of the intermediate solution was added to a vial.
In S7, lyophilization was performed to obtain a product, where the content of the BPA in the product was 4 g per vial.
In this comparative example, the lyophilization included pre-freezing, sublimation drying, and desorption drying.

The pre-freezing was maintaining the temperature at -50°C for 5 hours. The sublimation drying was maintaining the temperature at -20°C and the pressure at 10 Pa for 35 hours. The desorption drying was maintaining the temperature at 30°C and the pressure at 10 pa for 12 hours.

**Comparative Example 2**

Comparative Example 2 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 50 mg/mL of BPA.

In S5, the solution was sterilized by filtration through 0.45 micron and 0.22 micron filter membranes to obtain an intermediate solution.

In S6, filling was performed, i.e., 80 mL of the intermediate solution was added to a vial.

In S7, lyophilization was performed to obtain a product, where the content of the BPA in the product was 4 g per vial.

In this comparative example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

**Comparative Example 3**

Comparative Example 3 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 80 mg/mL of BPA.

In S5, the solution was sterilized by filtration through 0.45 micron and 0.22 micron filter membranes to obtain an intermediate solution.

In S6, filling was performed, i.e., 50 mL of the intermediate solution was added to a vial.

In S7, lyophilization was performed to obtain a product, where the content of the BPA in the product was 4 g per vial.

In this comparative example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

**Comparative Example 4**

Comparative Example 4 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 160 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 4 g per vial.

In this comparative example, the lyophilization included pre-freezing, sublimation drying, and desorption drying. The pre-freezing was maintaining the temperature at -10°C for 5 hours. The sublimation drying was maintaining the temperature at -5°C and the pressure at 10 Pa for 35 hours. The desorption drying was maintaining the temperature at 30°C and the pressure at 10 pa for 12 hours.

## Comparative Example 5

Comparative Example 5 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 40 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 1 g per vial.

In this comparative example, the lyophilization included pre-freezing, sublimation drying, and desorption drying. The pre-freezing was maintaining the temperature at -50°C for 5 hours. The sublimation drying was maintaining the temperature at -5°C and the pressure at 10 Pa for 35 hours. The desorption drying was maintaining the temperature at 30°C and the pressure at 10 pa for 12 hours.

## Comparative Example 6

Comparative Example 6 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 160 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 4 g per vial.

In this comparative example, the lyophilization included pre-freezing, sublimation drying, and desorption drying. The pre-freezing includes a first stage, a second stage, and a third stage. The first stage was maintaining the temperature at -50°C for 5 hours, the second stage was maintaining the temperature at -35°C for 3 hours, and the third stage was maintaining the temperature at -55°C for 4 hours. The sublimation drying was maintaining the temperature at -5°C and the pressure at 10 Pa for 28 hours. The desorption drying was maintaining the temperature at 30°C and the pressure at 10 pa for 12 hours. Reference may be made to Table 1.

## Comparative Example 7

Comparative Example 7 provided a preparation method for dihydroxyboronphenylalanine lyophilized powder for injection, including the following steps.

In S1, BPA and fructose were weighed and added to water for injection.

In S2, an appropriate amount of sodium hydroxide was added, followed by stirring until the solution was clear.

In S3, hydrochloric acid was added to adjust the pH to 7.5-8.5.

In S4, water was added to make the volume to obtain a solution containing 160 mg/mL of BPA.

In S5, the solution was sterilized by filtration through 0.45 micron and 0.22 micron filter membranes to obtain an intermediate solution.

In S6, filling was performed, i.e., 25 mL of the intermediate solution was added to a vial.

In S7, lyophilization was performed to obtain a product, where the content of the BPA in the product was 4 g per vial.

In this comparative example, the lyophilization included pre-freezing, sublimation drying, and desorption drying. The pre-freezing includes a first stage, a second stage, and a third stage. The first stage was maintaining the temperature at -50°C for 5 hours, the second stage was maintaining the temperature at -35°C for 3 hours, and the third stage was maintaining the temperature at -55°C for 4 hours. The sublimation drying was maintaining the temperature at -5°C and the pressure at 10 Pa for 35 hours. The desorption drying was maintaining the temperature at 30°C and the pressure at 10 pa for 12 hours. Reference may be made to Table 1.

# EP 4 678 166 A1

**Test Example 1**

In this test example, the lyophilized powders of Example 1 and Comparative Examples 6-7 of the present invention were studied, and some parameters of Example 1 and Comparative Examples 6-7 were shown in Table 1. The appearance of the lyophilized powder prepared in each of Example 1 and Comparative Examples 6-7 was observed, the moisture content and redissolution of the lyophilized powder were tested, and the result was recorded. The details are as shown in Table 2.

Table 1

| Item | | | Comparative Example 6 | Comparative Example 7 | Example 1 |
|---|---|---|---|---|---|
| BPA concentration | | | 160 mg/mL | 160 mg/mL | 160 mg/mL |
| Liquid volume | | | 25 mL | 25 mL | 25 mL |
| BPA content per vial | | | 4 g | 4 g | 4 g |
| Pre-freezing | First stage | Temperature /°C | -50 | -50 | -50 |
| | | Time /h | 5 | 5 | 5 |
| | Second stage | Temperature /°C | -35 | -35 | / |
| | | Time /h | 3 | 3 | / |
| | Third stage | Temperature /°C | -55 | -55 | / |
| | | Time /h | 4 | 4 | / |
| Sublimation drying | First stage | Temperature /°C | -5 | -5 | -5 |
| | | Time /h | 28 | 35 | 35 |
| | | Pressure /pa | 10 | 10 | 10 |
| Desorption drying | First stage | Temperature /°C | 30 | 30 | 30 |
| | | Time /h | 12 | 12 | 12 |
| | | Pressure /pa | 10 | 10 | 10 |
| Total lyophilization time /h | | | 52 | 59 | 52 |

Table 2

| Item | Comparative Example 6 | Comparative Example 7 | Example 1 |
|---|---|---|---|
| Appearance | The bottom was not fully dried | White loose lump | White loose lump |
| Redissolution time (s) | 180 | 60 | 60 |
| Moisture (%) | 4.87 | 0.58 | 0.57 |

With reference to Table 1 and Table 2, the lyophilized powder prepared in Example 1 of the present invention was a white loose lump with a redissolution time of 60 seconds and a moisture content of 0.57%, and had the advantages of short lyophilization time and full drying, indicating that the preparation method of the present invention could achieve an excellent lyophilization effect.

The pre-freezing in Comparative Example 6 and Comparative Example 7 adopted an annealing process. It can be found through comparison with Example 1 of the present invention that in Comparative Example 7, after the annealing process was adopted, the whole lyophilization process required a long time to achieve a lyophilization effect, and the lyophilized powder obtained was a white loose block, like that in Example 1 of the present invention; and in Comparative Example 6, after the annealing process was adopted, if the total lyophilization time of Example 1 of the present invention was adopted, complete lyophilization could not be achieved, the obtained lyophilized powder was not fully dried, the redissolution time was as long as 180 seconds, and the moisture content and as high as 4.87%.

Comparative Example 7 adopted an annealing process, but the appearance, redissolution time, and moisture content of the lyophilized powder prepared in Comparative Example 7 were substantially the same as those of the lyophilized powder prepared in Example 1 of the present invention, indicating that the annealing process in Comparative Example 7 did not provide a better effect, but had the disadvantages of long time and high costs.

It can be seen that the preparation method of the present invention can not only be used to prepare a large-specification lyophilized powder product with excellent properties, but also greatly shorten the total lyophilization time of the large-specification BPA, thereby improving production efficiency, reducing production costs, and expanding the range of applications.

**Test Example 2**

In this test example, the performance of the lyophilized powder prepared in Examples 1-20 and Comparative Examples 1-5 of the present invention was respectively studied, and some parameters of Examples 1-20 and Comparative Examples 1-5 were shown in Table 3. The appearance of the lyophilized powder prepared in each of Examples 1-20 and Comparative Examples 1-5 was observed, the moisture content and redissolution of the lyophilized powder were tested, and the result was recorded. The details are as shown in Table 4.

Table 3

| Item | BPA concentration | Liquid volume per vial | BPA content per vial | Lyophilization | | | | | | | | | Total time /h |
|---|---|---|---|---|---|---|---|---|---|---|---|---|---|
| | | | | Pre-freezing | | Sublimation drying | | | Desorption drying | | | | |
| | | | | Temperature /°C | Time /h | Temperature /°C | Time /h | Pressure /pa | Temperature /°C | Time /h | Pressure /pa | | |
| Example 1 | 160 mg/mL | 25 mL | 4 g | -50 | 5 | -5 | 35 | 10 | 30 | 12 | 10 | 52 |
| Example 2 | 200 mg/mL | 25 mL | 5 g | -50 | 5 | -5 | 35 | 10 | 30 | 12 | 10 | 52 |
| Example 3 | 200 mg/mL | 30 mL | 6 g | -50 | 5 | -5 | 35 | 10 | 30 | 12 | 10 | 52 |
| Example 4 | 250 mg/mL | 25 mL | 6.25 g | -50 | 5 | -5 | 35 | 10 | 30 | 12 | 10 | 52 |
| Example 5 | 160 mg/mL | 15 mL | 2.4 g | -50 | 5 | -5 | 35 | 10 | 30 | 12 | 10 | 52 |
| Example 6 | 160 mg/mL | 25 mL | 4 g | -60 | 5 | -5 | 35 | 10 | 30 | 12 | 10 | 52 |
| Example 7 | 160 mg/mL | 25 mL | 4 g | -40 | 5 | -5 | 35 | 10 | 30 | 12 | 10 | 52 |
| Example 8 | 160 mg/mL | 25 mL | 4 g | -30 | 5 | -5 | 35 | 10 | 30 | 12 | 10 | 52 |
| Example 9 | 160 mg/mL | 25 mL | 4 g | -20 | 5 | -5 | 35 | 10 | 30 | 12 | 10 | 52 |
| Example 10 | 160 mg/mL | 25 mL | 4 g | -50 | 2 | -5 | 35 | 10 | 30 | 12 | 10 | 49 |
| Example 11 | 160 mg/mL | 25 mL | 4 g | -50 | 10 | -5 | 35 | 10 | 30 | 12 | 10 | 57 |
| Example 12 | 160 mg/mL | 25 mL | 4 g | -50 | 5 | -10 | 35 | 10 | 30 | 12 | 10 | 52 |
| Example 13 | 160 mg/mL | 25 mL | 4 g | -50 | 5 | -2 | 35 | 10 | 30 | 12 | 10 | 52 |
| Example 14 | 160 mg/mL | 25 mL | 4 g | -50 | 5 | -5 | 24 | 10 | 30 | 12 | 10 | 41 |
| Example 15 | 160 mg/mL | 25 mL | 4 g | -50 | 5 | -5 | 54 | 10 | 30 | 12 | 10 | 71 |
| Example 16 | 160 mg/mL | 25 mL | 4 g | -50 | 5 | -5 | 30 | 10 | 30 | 12 | 10 | 47 |
| Example 17 | 160 mg/mL | 25 mL | 4 g | -50 | 5 | -5 | 35 | 10 | 25 | 12 | 10 | 52 |
| Example 18 | 160 mg/mL | 25 mL | 4 g | -50 | 5 | -5 | 35 | 10 | 35 | 12 | 10 | 52 |
| Example 19 | 160 mg/mL | 25 mL | 4 g | -50 | 5 | -5 | 35 | 10 | 30 | 5 | 10 | 45 |
| Example 20 | 160 mg/mL | 25 mL | 4 g | -50 | 5 | -5 | 35 | 10 | 30 | 14 | 10 | 54 |
| Comparative Example 1 | 160 mg/mL | 25 mL | 4 g | -50 | 5 | -20 | 35 | 10 | 30 | 12 | 10 | 52 |
| Comparative Example 2 | 50 mg/mL | 80 mL | 4 g | -50 | 5 | -5 | 35 | 10 | 30 | 12 | 10 | 52 |

(continued)

| Item | BPA concentration | Liquid volume per vial | BPA content per vial | Lyophilization | | | | | | | | | Total time /h |
|---|---|---|---|---|---|---|---|---|---|---|---|---|---|
| | | | | Pre-freezing | | Sublimation drying | | | Desorption drying | | | | |
| | | | | Temperature /°C | Time /h | Temperature /°C | Time /h | Pressure /pa | Temperature /°C | Time /h | Pressure /pa | | |
| Comparative Example 3 | 80 mg/mL | 50 mL | 4 g | -50 | 5 | -5 | 35 | 10 | 30 | 12 | 10 | | 52 |
| Comparative Example 4 | 160 mg/mL | 25 mL | 4 g | -10 | 5 | -5 | 35 | 10 | 30 | 12 | 10 | | 52 |
| Comparative Example 5 | 40 mg/mL | 25 mL | 1 g | -50 | 5 | -5 | 35 | 10 | 30 | 12 | 10 | | 52 |

Table 4

| Item | Appearance | Redissolution time | Moisture |
|---|---|---|---|
| Example 1 | White loose lump | 60s | 0.62% |
| Example 2 | White loose lump | 60s | 1.02% |
| Example 3 | White loose lump | 60s | 0.99% |
| Example 4 | White loose lump | 60s | 1.03% |
| Example 5 | White loose lump | 32s | 0.31% |
| Example 6 | White loose lump | 60s | 0.88% |
| Example 7 | White loose lump | 62s | 0.66% |
| Example 8 | Liquid spraying | 65s | 0.6% |
| Example 9 | Liquid spraying | 65s | 0.58% |
| Example 10 | White loose lump | 60s | 0.68% |
| Example 11 | White loose lump | 60s | 0.69% |
| Example 12 | White loose lump | 63s | 1.27% |
| Example 13 | White loose lump | 57s | 0.45% |
| Example 14 | White loose lump | 67s | 1.56% |
| Example 15 | White loose lump | 55s | 0.35% |
| Example 16 | White loose lump | 60s | 0.68% |
| Example 17 | White loose lump | 64s | 0.97% |
| Example 18 | White loose lump | 52s | 0.56% |
| Example 19 | White loose lump | 68s | 1.12% |
| Example 20 | White loose lump | 60s | 0.65% |
| Comparative Example 1 | The bottom was not completely dried and was light yellow in color | Failed to redissolve in 180s | 4.87% |
| Comparative Example 2 | 1/2 of the product was not completely dried and was light yellow in color | Failed to redissolve in 180s | 8.78% |
| Comparative Example 3 | 1/3 of the product was not completely dried and was light yellow in color | Failed to redissolve in 180s | 5.68% |
| Comparative Example 4 | Serious liquid spraying | 180s | 3.66% |
| Comparative Example 5 | Collapsed, incomplete lump structure | 180s | 2.18% |

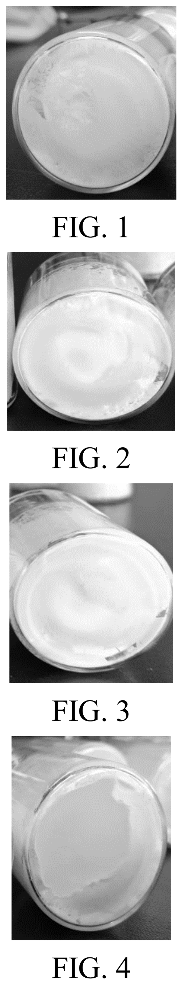

With reference to the contents of Table 3 and Table 4,
it can be seen according to Examples 1-5 that the preparation method of the present invention had an excellent lyophilization effect for high-concentration BPA, and could achieve a good technical effect with the process parameters within the concentration liquid volume ranges of the present invention. Specifically, as shown in FIG. 1, FIG. 2, and FIG. 3, FIG. 1 is an appearance diagram of the lyophilized powder prepared in Example 1, FIG. 2 is an appearance diagram of the lyophilized powder prepared in Example 2, and FIG. 3 is an appearance diagram of the lyophilized powder prepared in Example 3, where all of the lyophilized powders were white loose lumps. It can be seen that a lyophilized powder in the shape of a white loose lump, i.e., large-specification BPA injection, could be prepared by the preparation process of the present invention, the total lyophilization time was short, and a significant technical effect was achieved.

According to Example 1 and Examples 12-13, at the high sublimation drying temperature of -10°C to -2°C according to the present invention, high-concentration BPA could be effectively lyophilized, and the appearance, moisture content, and redissolution time of the lyophilized powder all met the application requirements. Moreover, the temperature could also improve the efficiency of sublimation drying and reduce the time of sublimation drying, thereby reducing costs. Referring to Example 12 and Example 13, the sublimation temperature in the range defined in the present invention could achieve a good technical result, and the higher the temperature, the higher the drying efficiency, and the lower the moisture content.

Compared with Examples 1, 12, and 13 of the present invention, in Comparative Example 1, the sublimation temperature was lower, the drying efficiency was lower, and when the sublimation in Comparative Example 1 lasted for the same time as that in Example 1, water could not be completely sublimated and the sublimation end point was not reached. Consequently, the bottom of the final product was not fully dried and was light yellow in color, and the product had a high moisture content of up to 4.87%, and finally could not fully redissolve in 180 seconds. Specifically, referring to FIG. 1 and FIG. 4, FIG. 1 is an appearance diagram of the lyophilized powder prepared in Example 1, which was a white loose lump; and FIG. 4 is an appearance diagram of the lyophilized powder prepared in Comparative Example 1, the bottom of which was not fully dried and was light yellow in color.

The preparation method of the present invention improved the efficiency of sublimation drying while achieving a drying effect, thereby shortening the overall lyophilization time and reducing costs. Generally, the sublimation drying temperature is low, because the sublimation critical temperature of the product needs to be lower than the collapse temperature, otherwise the lyophilized product will collapse, leading to a poor drying effect. As a result, generally a long time and high costs are required for fully drying the product by sublimation drying. Therefore, the preparation method of the present invention had a significant technical effect.

In Comparative Example 2, the liquid volume was 80 mL and the liquid height was about 80 mm. In Comparative Example 3, the liquid volume was 50 mL and the liquid height was about 50 mm. The higher the liquid height, the more difficult the product is to dry. In Comparative Example 2, 1/2 of the product was not completely dried and was light yellow in color. In Comparative Example 3, 1/3 of the product was not completely dried and was light yellow in color. The lyophilized powders prepared in Comparative Example 2 and Comparative Example 3 had a high final moisture content of up to 8.78%, and could not fully redissolve in 180 seconds. It can be found through comparing Example 1 with Comparative Examples 2 and 3 that the preparation method of the present application could realize a lyophilized powder for injection with high BPA content, and the lyophilized powder had excellent properties to meet application requirements.

In Examples 1, 6, and 7, the pre-freezing temperature was -60°C to -40°C, and the prepared lyophilized powder had a loose and lumpy appearance, with a low moisture content and short redissolution time. In Examples 8 and 9, the prepared lyophilized powder had a low moisture content and short redissolution time, but spraying of a small amount of liquid occurred. It can be seen that in the preparation method for large-specification BPA of the present invention, the freezing effect became worse as the pre-freezing temperature was increased, which led to the spraying of a small amount of liquid during subsequent sublimation.

In Comparative Example 4, the pre-freezing temperature was -10°C, which was too high. When the temperature was too high, the high-concentration BPA product could not be fully frozen, resulting in the formation of bubbles and serious liquid spraying during subsequent sublimation, and a long redissolution time. It can be seen that in the high-concentration BPA lyophilization process, when the pre-freezing temperature was higher than the pre-freezing temperature defined in the present invention, a significant product loss was caused, and a long time of about 180 seconds was taken to fully dissolve the drug on the wall of the vial.

In Comparative Example 5, the concentration of the lyophilization solution was 40 mg/mL, the lyophilization process in Example 1 of the present invention was adopted, and the prepared lyophilized powder collapsed in appearance, could not form a complete lump structure, and had a long dissolution time high moisture content. It is apparent that under the conditions of low concentration and high liquid volume as well as and the lyophilization conditions in the examples of the present invention, the lyophilization solution of Comparative Example 5 led to the product collapse phenomenon, affecting the lyophilization effect. It is conceivable that in the present invention, the use of the high-concentration lyophilization solution in combination with the lyophilization process of the present invention could not only reduce the collapse of the lyophilization solution, but also increase the drying efficiency and reduce the lyophilization costs.

According to Example 10 and Example 11, a good freezing effect could be achieved when the pre-freezing time was within the process range of the present invention. In addition, the overall pre-freezing time of the present invention was short, so that the overall lyophilization time could be effectively shortened and the costs could be reduced.

According to Example 14 to Example 16, a good drying effect could be achieved when the sublimation time was within the process range of the present invention. Provided that the temperature is constant, a good drying effect can be achieved with the time defined in the present invention. Provided that the temperature is constant, a longer time indicates a better drying effect and a lower moisture content, but increased costs. It is conceivable that in the present invention, the use of the sublimation drying temperature and holding time of the present invention in combination with pre-freezing can both achieve an excellent drying effect and further reduce lyophilization costs.

According to Example 17 to Example 20, a good drying effect could be achieved when the desorption temperature and the desorption time were respectively within the process ranges of the present invention. It is conceivable that provided that the desorption drying time is constant, a higher desorption drying temperature within the range defined in the present invention indicates higher drying efficiency and a lower moisture content; and provided that the desorption drying temperature is constant, a longer desorption drying time within the range defined in the present invention indicates a better drying effect and a lower moisture content, but increased costs. Since dihydroxyboronphenylalanine is thermally unstable, a desorption drying temperature exceeding the tolerance temperature of dihydroxyboronphenylalanine may lead to reaction of dihydroxyboronphenylalanine with a polyol, e.g., Maillard reaction of dihydroxyboronphenylalanine with fructose, resulting in a yellow appearance, and affecting the quality of the lyophilized powder.

In the examples of the present invention, the use of the lyophilization process of the present invention in combination with the high-concentration lyophilization solution can effectively shorten the overall lyophilization time, thereby improving the drying efficiency and overcoming the deficiencies in the prior art.

In the examples of the present invention, the lyophilized powder was tested by the following method.

① Appearance:
Inspection method: Visual inspection: The product was observed in natural light.
②Redissolution time:
Test method: A vial of the test sample was taken. An appropriate amount of purified water was added until the BPA concentration was 100 mg/mL. Timing was started while the vial was shaken. The timing was stopped after all the contents were dissolved. The redissolution time was recorded.
③ Moisture:

Drugs and reagents: Phosphorus pentoxide (analytical purity)
Instruments and equipment: vacuum drying oven, electronic balance (1/10000 precision)

A weighing bottle was taken, and dried under reduced pressure at 40°C to a constant weight with phosphorus pentoxide as a desiccant. The constant weight was recorded as $M_1$. 2 vials of the product were broken with a blunt instrument. The contents were taken out and mashed quickly. About 1 g of the mashed contents was taken and accurately weighed. The weight was recorded as $M_2$. The weighed mashed contents were placed in the constant-weight weighing bottle, dried under reduced pressure at 40°C for 24 hours, allowed to cool in the desiccator, and then weighed. The weight was recorded as $M_3$. The weight loss was calculated according to the following formula. Two parallel tests were carried out, and the average value was calculated. ①

$$\text{Loss on drying } (\%) = \frac{M_1 + M_2 - M_3}{M_2} \times 100\%$$

In the formula, $M_1$: weight of the dry weighing bottle, measured in g;

$M_2$: weight of the test sample, measured in g;

$M_3$: weight of the weighing bottle and the sample after drying, measured in g. ②

$$\text{Average weight loss } (\%) = \frac{\text{WeightLoss}_1 + \text{WeightLoss}_2}{2},$$

where $\text{WeightLoss}_1$ and $\text{WeightLoss}_2$ were respectively losses on drying (%) of two parallel tests.

**Test Example 3**

This test example studied the stability of the lyophilized powder prepared in Example 1 of the present invention, including the redissolution property, pH, related substances, and content of the lyophilized powder. Specifically, two batches of samples were prepared according to the method of Example 1, and the content and related substances of the samples were determined after the samples were respectively stored at 25°C for 0 and 3 months. The results are shown in Table 5 and Table 6.

The test method of the present invention may be as follows.
① Property: Inspection method: Visual inspection. Specific test operation: The product was observed in natural light.
② pH: Test method: pH determination method (Chinese Pharmacopoeia 2015 edition, Part IV, General Rules 0631).
③ Related substances: HPLC method (Chinese Pharmacopoeia 2015 edition, Part IV, General Rules 0512).
④ Content: HPLC method (Chinese Pharmacopoeia 2015 edition, Part IV, General Rules 0512). The BPA content was determined in this test example.

Table 5

| Test item | | 25°C | |
|---|---|---|---|
| | | Month 0 | Month 3 |
| Property | | White loose lump | White loose lump |
| pH | | 8.00 | 7.94 |
| Related substances (%) | L-tyrosine | 0.12 | 0.12 |
| | L-phenylalanine | 0.08 | 0.09 |
| | Other maximum single impurity | Not detected | 0.01 |
| | Total impurities | 0.20 | 0.21 |
| Content (%) | | 102.1 | 102.9 |

Table 6

| Test item | | 25°C | |
|---|---|---|---|
| | | Month 0 | Month 3 |
| Property | | White loose lump | White loose lump |
| pH | | 8.10 | 8.10 |
| Related substances (%) | L-tyrosine | 0.10 | 0.11 |
| | L-phenylalanine | 0.09 | 0.08 |
| | Other maximum single impurity | 0.01 | 0.01 |
| | Total impurities | 0.20 | 0.19 |
| Content (%) | | 101.3 | 102.5 |

It can be known from Table 5 and Table 6, after storage at 25°C for 3 months, the properties and pH of the lyophilized powder were very stable, the impurity content of related substances was also very small, and the content of active ingredient BPA was also stable, meeting the usage requirements. It can be seen that the two batches of samples prepared in Example 1 of the present invention had good stability after being stored at 25°C for 3 months. It can be seen that the large-specification lyophilized powder prepared by the preparation method of the present invention has excellent stability and can meet the usage requirements.

To sum up, the large-specification dihydroxyboronphenylalanine lyophilized powder for injection prepared by the preparation method of the present invention has a loose appearance and very low moisture content and meets the requirements of use and storage, indicating an excellent lyophilization effect. Moreover, the redissolution time and stability of the lyophilized powder are excellent and meet the usage requirements. In addition, the preparation method of the present invention can greatly shorten the overall lyophilization time of the large-specification dihydroxyboronphenylalanine, thereby improving the production efficiency, reducing the production costs, and providing high economic value.

The foregoing descriptions are exemplary implementations of the present invention. It is noted that a person of ordinary skill in the art may make some improvements and modifications without departing from the principle of the present invention and the improvements and modifications shall fall within the protection scope of the present invention.

## Claims

1. A preparation method for large-specification dihydroxyboronphenylalanine lyophilized powder for injection, comprising:

mixing dihydroxyboronphenylalanine, a polyol, a base, and part of a solvent to obtain a first mixed solution;
adjusting the pH of the first mixed solution to 7.5-8.5 by using a pH regulator, and adding the remaining part of the solvent to obtain a second mixed solution, wherein the concentration of dihydroxyboronphenylalanine in the second mixed solution is 160-250 mg/mL; and
aliquoting the second mixed solution, followed by lyophilization, wherein the lyophilization comprises pre-freezing, sublimation drying, and desorption drying.

2. The preparation method according to claim 1, wherein conditions of the pre-freezing comprise: a temperature of -60°C to -20°C and atmospheric pressure.

3. The preparation method according to claim 1, wherein conditions of the sublimation drying comprise: a temperature of -10°C to -2°C and a pressure of 10-20 pa.

4. The preparation method according to claim 1, wherein conditions of the desorption drying comprise: a temperature of 25°C to 35°C and a pressure of 10-20 pa.

5. The preparation method according to claim 1~4, wherein the time of the pre-freezing is 2-10 hours.

6. The preparation method according to claim 5, wherein the pre-freezing is: maintaining the temperature at -50°C for 2-10 hours.

7. The preparation method according to claim 1~4, wherein the time of the sublimation drying is 24-54 hours.

8. The preparation method according to claim 7, wherein the sublimation drying is: maintaining the temperature at -5°C for 24-54 hours.

9. The preparation method according to claim 1~4, wherein the time of the desorption drying is 5-15 hours.

10. The preparation method according to claim 1, wherein the mass ratio of the dihydroxyboronphenylalanine to the polyol is 1:1 to 1:1.3; the polyol is selected from at least one of fructose, sorbitol, mannitol, erythritol, maltitol, lactitol, or xylitol.

11. The preparation method according to claim 1, wherein the base is selected from at least one of sodium hydroxide, magnesium hydroxide, potassium hydroxide, or calcium hydroxide; and/or

    the pH regulator is selected from at least one of hydrochloric acid, acetic acid, phosphoric acid, nitric acid, or sulfuric acid; and/or
    the solvent is water for injection.

12. The preparation method according to claim 1, wherein the second mixed solution is subjected to sterilization treatment before the lyophilization; and the sterilization treatment comprises: sterilizing the second mixed solution by filtration through a microfiltration membrane.

13. The preparation method according to claim 1, wherein the liquid volume in each vial after the aliquoting is 15-30 mL.

14. The preparation method according to claim 1, wherein the liquid height is 15-30 mm after aliquoting.

15. The preparation method according to claim 1, wherein each vial obtained after the aliquoting contains 3-7.5 g of dihydroxyboronphenylalanine.

16. A lyophilized powder for injection, prepared by the preparation method according to any one of claims 1 to 15.

17. The lyophilized powder for injection according to claim 16, wherein each vial of the lyophilized powder for injection contains 3-7.5 g of dihydroxyboronphenylalanine.

FIG. 1

FIG. 2

FIG. 3

FIG. 4

## INTERNATIONAL SEARCH REPORT

| International application No. |
|---|
| **PCT/CN2024/080013** |

| A. | CLASSIFICATION OF SUBJECT MATTER |
|---|---|
| | A61K 9/19(2006.01)i; A61K 41/00(2020.01)i; A61K 47/26(2006.01)i; A61K 47/02(2006.01)i; A61P 35/00(2006.01)i |

According to International Patent Classification (IPC) or to both national classification and IPC

| B. | FIELDS SEARCHED |
|---|---|

Minimum documentation searched (classification system followed by classification symbols)

IPC:A61K A61P

Documentation searched other than minimum documentation to the extent that such documents are included in the fields searched

Electronic data base consulted during the international search (name of data base and, where practicable, search terms used)

CNABS; NCBI; JPABS; TWABS; CNTXT; WOTXT; USTXT; EPTXT; CJFD; CNKI; VEN; DWPI; SIPOABS; PUBMED; ELSEVIER; Patentics; STNext; 万方, WANFANG; 读秀, DUXIU: 二羟硼基苯丙氨酸, 硼化苯丙氨酸, 对二羟苯丙氨酸硼, 二羟基硼基苯丙氨酸, BPA, 76410-58-7, 冻干, 多元醇, 果糖, 山梨糖醇, 甘露糖醇, 赤藓糖醇, 麦芽糖醇, 乳糖醇, 木糖醇, 碱, 氢氧化, 酸, PH, freeze-dried, polyol, Fructose, sorbitol, mannitol, erythritol, maltitol, lactitol, xylitol

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 111920964 A (NEUBORON MEDTECH LTD.) 13 November 2020 (2020-11-13) description, paragraphs [0006]-[0020] | 1, 2, 4-7, 9-17 |
| A | CN 111920964 A (NEUBORON MEDTECH LTD.) 13 November 2020 (2020-11-13) description, paragraphs [0006]-[0020] | 3, 8 |
| A | WO 2004030661 A2 (PSIMEI PHARMACEUTICALS PLC et al.) 15 April 2004 (2004-04-15) embodiment 3 | 1-17 |
| A | CN 113559261 A (DONGGUAN DONGYANGGUANG BORON MEDICINE RESEARCH AND DEVELOPMENT CO., LTD.) 29 October 2021 (2021-10-29) embodiment 1 | 1-17 |

☐ Further documents are listed in the continuation of Box C.          ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 June 2024** | **11 June 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/080013**

| Patent document cited in search report | | | Publication date (day/month/year) | Patent family member(s) | | | Publication date (day/month/year) |
|---|---|---|---|---|---|---|---|
| CN | 111920964 | A | 13 November 2020 | WO | 2020216334 | A1 | 29 October 2020 |
| | | | | EP | 3960204 | A1 | 02 March 2022 |
| | | | | EP | 3960204 | A4 | 06 July 2022 |
| | | | | US | 2021338588 | A1 | 04 November 2021 |
| | | | | JP | 2022519619 | A | 24 March 2022 |
| | | | | JP | 7297077 | B2 | 23 June 2023 |
| WO | 2004030661 | A2 | 15 April 2004 | WO | 2004030661 | A3 | 30 September 2004 |
| | | | | AU | 2003260793 | A1 | 23 April 2004 |
| | | | | GB | 0222945 | D0 | 13 November 2002 |
| CN | 113559261 | A | 29 October 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103100094 B
- CN 113546048 A